(19) Europäisches Patentamt
European Patent Office
Office européen des brevets

(11) Publication number: **0 249 950 B1**

# (12) EUROPEAN PATENT SPECIFICATION

(45) Date of publication of patent specification: **19.06.91**

(51) Int. Cl.5: **C07D 213/54**, C07D 213/55

(21) Application number: **87108671.6**

(22) Date of filing: **03.02.83**

(60) Publication number of the earlier application in accordance with Art.76 EPC: **0 085 959**

## (54) Aromatic compounds.

(30) Priority: **04.02.82 GB 8203261**
**18.10.82 GB 8229705**

(43) Date of publication of application:
**23.12.87 Bulletin 87/52**

(45) Publication of the grant of the patent:
**19.06.91 Bulletin 91/25**

(84) Designated Contracting States:
**AT BE CH DE FR GB IT LI LU NL SE**

(56) References cited:
**FR-A- 2 197 583**

**THE JOURNAL OF THE ORGANIC CHEMISTRY, vol. 35, no. 12, Decembber 1970; pages 4096-4100, Baltimore, US; R.G. PEWS et al.: "The cycloaddition of aroyl and sulfonyl cyanides with 5,5-Dimethoxy-1,2,3,4-tetrahalocyclopentadienes"**

(73) Proprietor: **THE WELLCOME FOUNDATION LIMITED**
**Unicorn House 160 Euston Road**
**London NW1 2BP(GB)**

(72) Inventor: **Coker, Geoffrey George**
**80 Pickhurst Park**
**Bromley, Kent(GB)**
Inventor: **Findlay, John William Addison**
**Rt. 2, Box 514, Cascade Drive**
**Chapel Hill, NC 27514(US)**

(74) Representative: **Dipl.-Ing. Schwabe, Dr. Dr. Sandmair, Dr. Marx**
**Stuntzstrasse 16**
**W-8000 München 80(DE)**

**EP 0 249 950 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a group of novel compounds useful for the preparation of certain pyridyl derivatives exhibiting anti-histamine activity, which are the subject-matter of European Patent 0 085 959.

US Patent No. 2567245 discloses a group of pyridyl aliphatic amines with antihistamine activity and specifically discloses 3-(p-bromophenyl)-3-(2-pyridyl)-N,N-dimethylpropylamine and 3-(p-chlorophenyl)-3-(2-pyridyl)-N,N-dimethyl-propyl-amine which are hereinafter referred to by their generic names brompheniramine and chlorpheniramine, respectively.

US Patent 2,712,023 discloses a group of pyridyl propenylamines with antihistamine activity, the most outstanding of which is the compound named (E)-1-(4-methyl-phenyl)-1-(2-pyridyl)-3-pyrrolidinoprop-1-ene and hereinafter referred to by its generic name, triprolidine. Triprolidine has gained widespread clinical acceptance and is one of the most potent antihistamines available.

Triprolidine is known to be metabolized in man to (E)-1-(4-carboxyphenyl)-1-(2-pyridyl)-3-pyrrolidinoprop-1-ene which has little or no antihistamine activity.

The antihistamines now in use, including diphenylhydramine, the pheniramines, pyrilamine, promethazine and triprolidine have one potential disadvantage in common; they all cause sedation or drowsiness in some patients.

European Patent 0 085 959 from which the present application is a divisional relates to a novel group of compounds having antihistamine activity which are compounds of the formula (I).

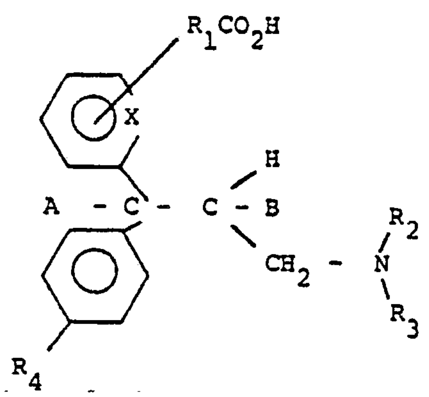

(I)

or a salt, ester or amide thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken together with the nitrogen comprise a nitrogen-containing heterocyclic ring having four to six ring members;

$R_4$ is hydrogen, halogen, hydroxy, cyano, $C_{1-4}$acyloxy, $C_{1-4}$ alkoxy or $C_{1-4}$ alkyl optionally substituted by one to three halogen atoms;

X is -N= or -CH=; and

A and B each represent hydrogen atoms or -CA-CB- represents -C=C-.

Of the compounds of formula (I) those of formula (II) are preferred.

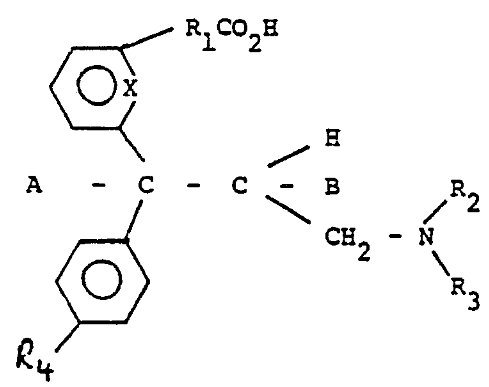

(II)

or a salt, ester or amide thereof; wherein $R_1$ to $R_4$, X, A and B are as defined in relation to formula (I).

$R_1$ may be a straight or branched chain, saturated or unsaturated hydrocarbon group or a single bond. Suitably $R_1$ is a straight chain hydrocarbon group or a single bond. Suitably $R_1$ contains at the most one double or triple bond. Preferably $R_1$ is a group $(CH_2)_n$ wherein n is an integer 0 to 7, or a group $(CH_2)_a CH=CH(CH_2)_b$ where a and b are independently 0 to 5 and the sum of a and b does not exceed 5.

Further preferred compounds of the formula (I) include those of the formula (IIIa) or (IIIb)

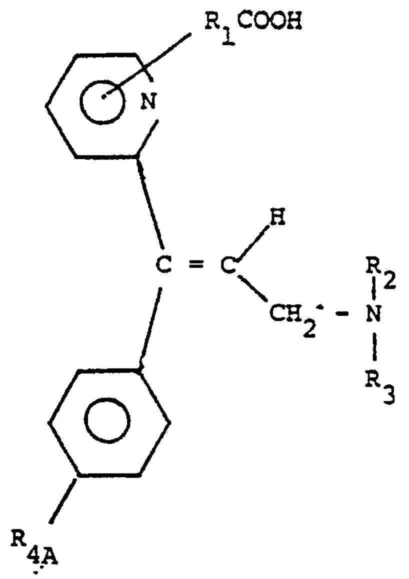

(IIIa)

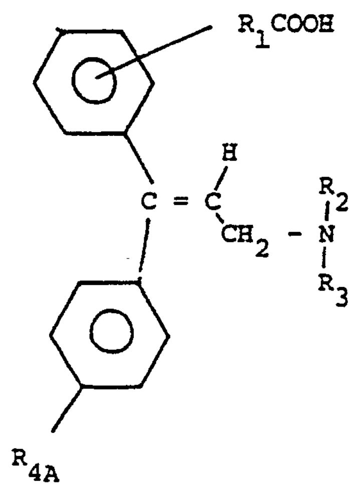

(IIIb)

or a salt, ester or amide thereof;
wherein $R_1$ is $(CH_2)_n$, where n is an integer 1 to 7, or $(CH_2)_aCH:CH(CH_2)_b$, where a and b are independently 0 to 5 and the sum of a and b does not exceed 5; $R_2$ and $R_3$ are the same or different and are hydrogen, lower alkyl (1 to 4 carbons) or taken together with the nitrogen comprise a nitrogen-containing heterocyclic ring (of four to six ring members) such as pyrrolidino, piperidino or morpholino; and $R_{4A}$ is hydrogen, halogen lower alkyl (1 to 4 carbons) or lower alkoxy (1 to 4 carbons).

Suitably n is 0 to 3 and preferably n is 2. Suitably the sum of a and b does not exceed 2 and preferably a and b are both 0.

Suitably $R_2$ and $R_3$ are the same or different and each is methyl or ethyl or taken together with the nitrogen atom to which they are attached form a four to six membered heterocyclic ring, preferably a saturated heterocyclic ring such as pyrrolidine, piperidine or morpholine. $NR_2R_3$ is preferably a pyrrolidino group or a dimethylamino group.

Suitably $R_4$ is hydrogen, halogen, $C_{1-4}$ alkyl, $C_{1-4}$ alkoxy or trifluoromethyl. Most suitably $R_4$ is hydrogen, methyl, ethyl, trifluoromethyl, methoxy, bromo, chloro or fluoro. Preferably $R_4$ is methyl, trifluoromethyl, methoxy, bromo or chloro. Most preferably $R_4$ is methyl.

Preferably X is -N=

A preferred group of compounds of the formula (I) is that of the formula (IV):

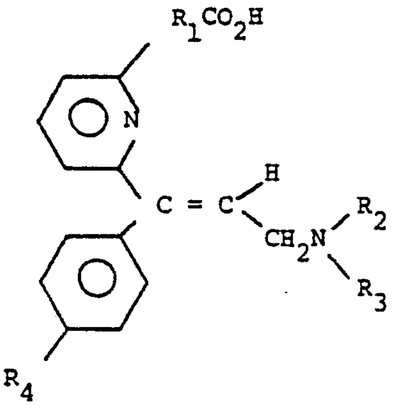

(IV)

or a salt, ester or amide thereof;

wherein $R_1$ to $R_4$ are as hereinbefore defined. Of the compounds of the formula (IV), those wherein $R_1$ is a single bond (i.e. n=O), CH=CH or $CH_2CH_2$, $NR_2R_3$ is pyrrolidino and $R_4$ is methyl or trifluoromethyl are particularly preferred.

A further preferred group of compounds of the formula (I) is that of the formula (V)

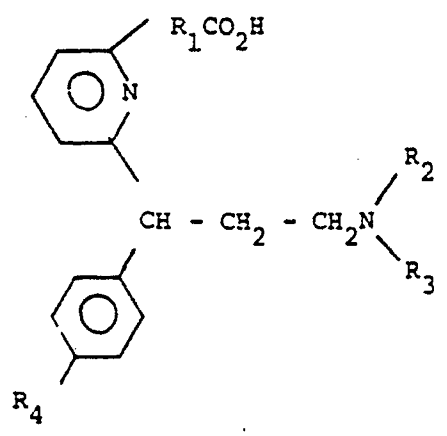

(V)

or a salt, ester or amide thereof; wherein $R_1$ to $R_4$ are as hereinbefore defined. Of the compounds of the formula (V), those wherein $R_1$ is a single bond, CH=CH or $CH_2CH_2$, $NR_2R_3$ is dimethylamino and $R_4$ is chlorine or bromine are particularly preferred.

Amides of the compounds of the formula (I) are amides conventionally formed from carboxylic acids. Amides formed from ammonia, primary amines or amino acid, such as glycine, are particularly suitable.

Solvates of the compounds of the formula (I) are also included. Preferred solvates include hydrates and $C_{1-4}$ alkanolates.

When the compounds of formula (I) contain a double bond in the side chain terminating in the group $NR_2R_3$, for example the compounds of formula (IV), they exist in either the cis or trans isomeric form(s) (in relation to the X-containing ring). The compounds of the formula (IV) have been drawn in the trans configuration and these are the isomers which primarily have useful antihistamine activity. The compounds in the cis configuration are primarily useful as intermediates in preparing the trans isomers. There are also provided mixtures of the isomers. When $R_1$ in the substituent $R_1CO_2H$ contains a double bond, further isomers of the compounds of the formula (I) exist, and both isomers and the isomeric mixture of these compounds are included. When $R_1CO_2H$ contains a double bond, the preferred isomers are those wherein the carboxylic acid group is trans to the aromatic ring.

Esters and amides of the compounds of the formula (I) whilst having some antihistamine activity in their own right may also be useful intermediates in the preparation of the carboxy compounds of the formula (I). Suitable esters include conventional ester groups known to be useful for protecting carboxylic acid groups such as $C_{1-6}$ alkyl esters wherein the alkyl group is straight or branched chain and is optionally substituted

by halogen. Alkyl esters ($C_{1-4}$) are particularly preferred.

Salts of the compounds of formula (I) may be either acid addition salts or salts formed with the carboxylic acid group. Acid addition salts are preferred but salts formed from the carboxylic acid group may be particularly useful in preparing the corresponding carboxy compound. Pharmaceutically acceptable salts are preferred.

There are also provided methods for preparing compounds of the formula (I), which methods include:

a) The reaction of a compound of the formula (VI)

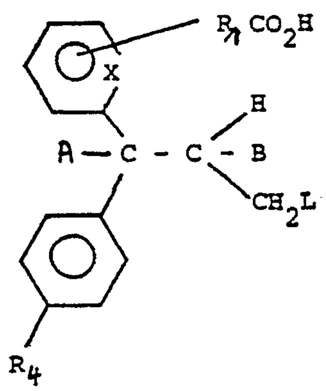

. (VI)

or an ester thereof with an amine $HNR_2R_3$ wherein X, A, B and $R_1$ to $R_4$ are as hereinbefore defined and L is a leaving group; and

b) When it is required to prepare a compound of the formula (I) wherein CA-CB represents a double bond:

The reaction of an ester, amide or carboxylic acid salt of a compound of the formula (IX):

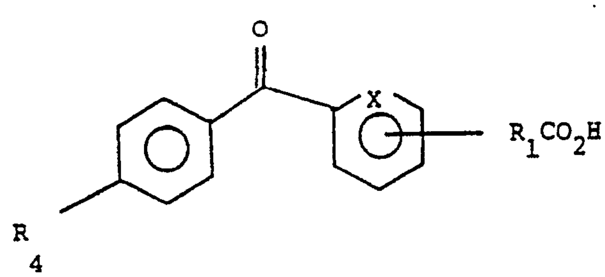

(IX)

with a Wittig reagent suitable for attaching the side chain $=CHCH_2NR_2R_3$ wherein, X and $R_1$ to $R_4$ are as hereinbefore defined, followed by deprotection of the carboxy group if desired;

The present invention relates to novel compounds of Formula (IXa)

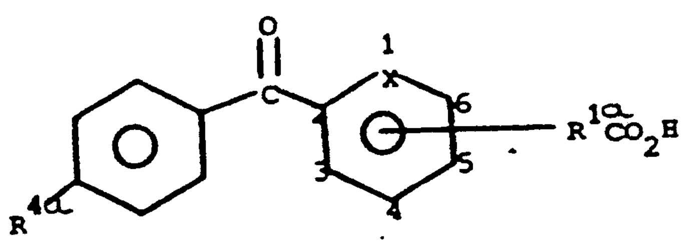

(IXa)

or a salt, ester, or amide thereof, wherein X is nitrogen; the group $R^{1a}CO_2H$ is attached to the 6-position of the pyridine ring; $R^{1a}$ is $(CH_2)_n$ wherein n is an integer 1 to 7, or $(CH_2)_aCH=CH(CH_2)_b$ wherein a and b are independently 0 to 5 and the sum of a and b does not exceed 5; and $R^{4a}$ is hydrogen, halogen, $C_{1-4}$ alkyl, $C_{1-4}$ alkoxy or trifluoromethyl. The compounds are useful intermediates for the preparation of compounds of Formula (I), for example according to process (b) above. The intermediates are also useful in the preparation of compounds (VI) employed in process (a), as described in more detail below.

a) Suitable leaving groups L in the compounds of the formula (VI) are those as defined by J.March, Advanced Organic Chemistry, 2nd ed., pages 683 and 895, McGraw Hill, New York, 1977, e.g. -Br, -Cl, toluene sulphonate, methane sulphonate, acyloxy (such as acetate), etc.

This reaction will normally be carried out in a solvent suitable for carrying out such displacement reactions, for example a polar solvent, such as a $C_{1-4}$ alkanol or a polar aprotic solvent such as DMSO, at a temperature between 0 and 180° C.

The compounds of the formula (VI) may be prepared by the reaction of an ester of the corresponding compound where L is a hydroxy group with an acid or a suitable reactive acid derivative, followed by removal of the ester function if desired. Suitable reactants include hydrogen halides, halogenated phosphorus compounds such as phosphorus pentachloride or phosphorus oxychloride, a suitable sulphonyl chloride (such as methane sulphonyl chloride or p-toluene sulphonyl chloride) or an acid anhydride, such as acetic anhydride. The reaction will conveniently be carried out in a suitable solvent under conditions well known to those skilled in the art, for example a non-protic solvent such as an ether or a halogenated hydrocarbon, in the presence of a base such as a tertiary amine (for example triethylamine) at a non-extreme temperature, for example between 0° and 100° C and conveniently at room temperature. When a tertiary amine is used as a base, an excess of this may be used as the solvent.

The hydroxy compounds may be prepared by the reaction of a compound of formula (IX) with an appropriate Wittig reagent containing a protected hydroxy group for example

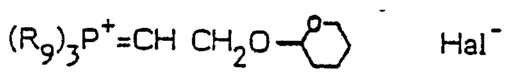

wherein $R_9$ is a $C_{1-4}$ alkyl or phenyl group, which is liberated by the action of strong base on the corresponding phosphonium salt

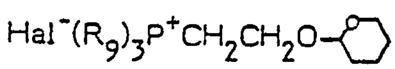

where Hal is chlorine or bromine.

b) This reaction is a conventional Wittig reaction and, as such, is analogous to those described in Organic Reactions, 14, 270-490 (1965) and Pure and Applied Chemistry, 9, 245-254 (1964). The reaction is suitably carried out in an anhydrous solvent inert under the reaction conditions utilised, for example toluene, benzene, tetrahydrofuran, dioxan, glycol ethers and $C_{1-6}$ alkyl ethers such as ethyl ether, at a temperature between -80° C and 100° C. The Wittig reagent will normally be prepared by treatment of a phosphonium salt with a strong base, for example a $C_{1-4}$ alkyl or aryl lithium compound such as butyl lithium, or a metal hydride, such as sodium hydride in a suitable inert solvent, for example an ether such as tetrahydrofuran.

The Wittig reagent in reaction (b) is conveniently a compound of the formula $(R_9)_3P=CHCH_2NR^2R^3$ which can be liberated from its corresponding phosphonium salt $(R_9)_3P^+CH_2CH_2NR_2R_3$ $Hal^-$ wherein Hal, $R_2$ and $R_3$ are as hereinbefore defined and $R_9$ is a $C_{1-4}$ alkyl or phenyl group by reaction with a strong base. The reaction is suitably carried out in an inert solvent such as toluene or tetrahydrofuran at a temperature of between 0° and 50° C and conveniently at room temperature. Suitably the strong base is an alkyl or aryl lithium compound, such as butyl lithium, or a metal hydride, such as sodium hydride. The use of butyl lithium in toluene at room temperature has been found to be particularly convenient. The phosphonium salts $(R_9)_3P^+CH_2CH_2NR_2R_3$ $Hal^-$ may be prepared by known methods (see, for example, UK Patent No.1161201).

Compounds of formula (IXa) in which $R^{1a}$ is -CH=CH- (trans) may be prepared by reacting a compound of formula (XV) with an acrylate ester (XVI) in the presence of a catalyst consisting of palladium acetate and a triarylphosphine and a tertiary amine such as triethylamine or tributylamine at an elevated temperature, for example 120° to 180° C, conveniently 140° to 150°. The reaction may be carried out under pressure to achieve the desired temperature range if desired. Optionally a solvent such as acetonitrile may be used and the reactants may be heated together in a sealed pressure vessel (e.g see R.F.Heck et al., J. Org. Chem., 43, 2947 (1978)).

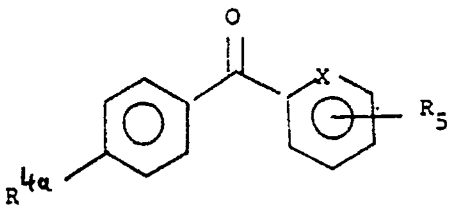

(XV)

$CH_2 = CHCO_2R_9$ (XVI)

wherein $R^{4a}$ is as defined above, $R_5$ is a halogen atom, and $R_9$ is a $C_{1-4}$ alkyl group.

Compounds of formula (IXa) may also be prepared by reacting a compound of formula (XVII):

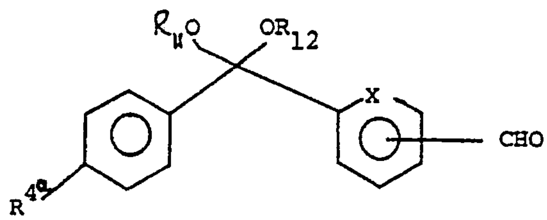

(XVII)

.

wherein $R_{11}$ and $R_{12}$ may be the same or different and are each $C_{1-4}$ alkyl, or may together form a cyclic ketal containing up to 6 carbon atoms, with malonic acid in the presence of a suitable base such as as pyridine or piperidine, or with a Wittig reagent prepared by treating a phosphonium salt (XVIII A) or a phosphonate ester (XVIII B) with a suitable base in an appropriate solvent:

$(R_9)_3P^+(CH_2)_dCO_2R_6Hal^-$ (XVIII A)

$(R_{10})_2PO(CH_2)_dCO_2R_6$ (XVIII B)

wherein Hal, $R_6$, $R_9$, $R_{10}$ and d are as defined above. The ketone (IXa) is generated by acidic hydrolysis of the protecting ketal. The double bond in the group $R^{1a}$ may be reduced if desired with hydrogen in presence of a catalyst such as palladium charcoal.

Compounds of formula (XVII) may be prepared from compounds of formula (XV) by conversion to a ketal by reaction with a mono or dihydroxy compound in the presence of an acid catalyst followed by reaction with a metal alkyl compound, for example butyllithium, and subsequent treatment with dimethylformamide. The reaction is preferably conducted at low temperature (below -60 °C) in a solvent such as toluene.

In turn compounds of formula (XV) can be prepared by treatment of a compound of formula (XVIII) with a metal alkyl compound, for example butyllithium, in a suitable solvent such as toluene, followed by reaction with a compound of formula (XIX) wherein $R_5$ is halogen such as chlorine or bromine and $R^{4a}$ is as hereinbefore defined.

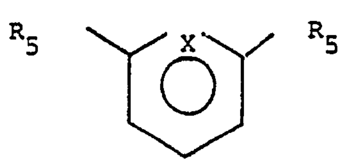

(XVIII)

.

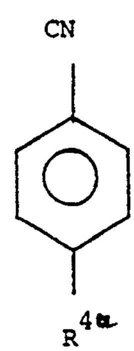

(XIX)

The following Examples are provided by the way of illustration of the present invention. All temperatures indicated are in degrees Celsius.

Example 1: (E)-3-(6-(3-Pyrrolidino-1-(4-tolyl)prop-1E-enyl(-2-pyridyl)acrylic acid (Compound A) via the intermediate Ethyl-(E)-3-(6-(4-toluyl)-2-pyridyl)-acrylate (compound 2).

Butyllithium (50 mL, 1.65M in hexane) was added under nitrogen to a stirred suspension of 2,6-dibromopyridine (19.5 g) in dry ether (200 mL) at -50°. After 0.75 hr a solution of 4-tolunitrile (10 g) in ether (50 mL) was added; stirring was continued at -50° for 3 hrs. The mixture was allowed to warm to -30° and treated with hydrochloric acid (200 mL, 2M). The precipitated solid was collected, washed with water, and recrystallized from aqueous ethanol. The 2-bromo-6-(4-toluoyl)pyridine formed colourless needles (12.2 g) m.p. 97-98°.

A mixture of 2-bromo-6-(4-toluoyl)pyridine (200 g), ethylene glycol (85 mL), p-toluenesulphonic acid (32 g) and benzene (11 mL) was boiled under a Dean/Stark trap until water collection had become very slow (about 20 mL collected in 16 hours).

The cooled solution was poured into ice/water containing sodium carbonate (100 g) with stirring. The benzene layer was separated, washed with water, dried with sodium sulphate and evaporated to about 500 mL. Cooling gave a first crop of 2-(6-bromo-2-pyridyl)-2-(4-tolyl)-1,3-dioxolan (compound 1), m.p. 113-114° (170 g). Dilution with petroleum ether gave a second crop, m.p. 109-112° (34 g). The residue after evaporation (31 g) was recycled.

A solution of compound 1, vide supra, (70 g) in dry toluene (800 mL) was added dropwise during 5 hr to a stirred solution of butyllithium (1.6M in hexane, 200 mL) and toluene (200 mL) at -65 to -72° under nitrogen. After a further 30 minutes at -70°, dry dimethylformamide (40 mL) was added during 35 minutes. Stirring continued overnight at -70 to -60°.

Hydrochloric acid (2N, 400 mL) was added, allowing the temperature to rise to about -10°. After 30 minutes, 2N ammonia (ca. 90 mL) was added to pH 7-8. The toluene layer was separated and the aqueous phase was extracted with ether. The combined organic liquids were washed with ice/water, dried ($MgSO_4$) and evaporated in vacuo below 50°. The aldehyde, 2-(6-formyl-2-pyridyl)-2-(4-tolyl)-1,3-dioxolan, (63.9 g) crystallized on keeping at 3°, m.p. 52-63°.

The aldehyde prepared above (2.5 g) was dissolved in 1,2-dimethoxyethane (10 mL) and added to a solution of the phosphonate carbanion produced from triethyl phosphonoacetate (2 g) and sodium hydride (0.22 g) in the same solvent. The mixture was stirred for two hours, diluted with ether (25 mL) and treated with hydrochloric acid (5 mL, 2M). The organic phase was separated, washed with water, dried, and evaporated. The resulting oil was dissolved in ethanol (20 mL) containing concentrated hydrochloric acid (3 mL) and water (3 mL). After heating on the steam bath for ten minutes, the solution was diluted with ice water, rendered alkaline with sodium bicarbonate solution, and extracted with ether. Evaporation gave **Ethyl-(E)-3-(6-(4-toluyl)-2-pyridyl)acrylate (compound 2)** which crystallized from cyclohexane in colourless platelets (1 g), m.p. 108-111°.

Butyllithium (10 mL, 1.64M in hexane) was added under nitrogen to a stirred suspension of triphenyl-2-pyrrolidinoethylphosphonium bromide (7.2 g) in dry toluene (75 mL). After 0.5 hr, compound 2, vide supra, - (4.8 g) in toluene (50 mL) was added. The suspension, initially orange, became deep purple, then slowly faded to yellow during 2 hours heating at 75°. The cooled solution was diluted with ether (150 mL) and treated with hydrochloric acid (50 mL, 2M). The aqueous phase was separated, washed with ether, and basified with potassium carbonate (ice) and extracted with ether. The mixture of isomeric esters obtained by evaporation was dissolved in ethanol (100 mL) containing sodium hydroxide solution (20 mL, 1M) and partially evaporated on the steam bath under reduced pressure for 5 minutes. The residual aqueous solution was neutralized with sulphuric acid (20 mL, 0.5M) and evaporated to dryness. The solid residue was extracted with hot isopropanol (3 x 50 mL) and the extracts were concentrated until crystallization commenced. The (E)-3-(6-(3-pyrrolidino-1-(4-tolyl)prop-1E-enyl)-2-pyridyl)acrylic acid (Compound A), after recrystallization from isopropanol, melted at 222° (decomp).

Example 2: 3-(6-(3-Pyrrolidino-1-(4-tolyl)prop-1E-enyl)-2-pyridyl)propionic acid. (Compound B) via the intermediate Ethyl-(E)-3-(6-(4toluyl)-2-pyridyl)-acrylate (compound 2).

A solution of compound 2, vide supra, (3 g) in alcohol (100 mL) containing Raney nickel (1 g) was stirred under hydrogen at room temperature and pressure until the calculated quantity of hydrogen had been absorbed (ca. 45 minutes). The reduced ester was recovered by filtration and evaporation and purified by column chromatography on silica gel using petroleum ether as eluent. Treatment of this ester with Wittig

reagent by the method of Example 1 followed by saponification gave a mixture of two isomeric acids which were separated by fractional crystallization from ethyl acetate/petroleum ether mixtures. The less soluble E isomer, 3-(6-(3-pyrrolidino-1-(4-tolyl)prop-1E-enyl)-2-pyridyl)propionic acid (Compound B), melted at 156-7°.

Example 3: (E)-3-(6-(3-Dimethylamino-1-(4-tolyl)prop-1E-enyl)-2-pyridyl)acrylic acid (Compound C) via the intermediate Ethyl-(E)-3-(6-(4-toluyl-) 2-pyridyl)-acrylate (compound 2).

Treatment of compound 2, vide supra, with the Wittig reagent derived from triphenyldimethylaminoethylphosphonium bromide by the method of Example 1 gave a mixture of isomeric acids which were separated by fractional crystallization from ethyl acetate. The less soluble E-isomer, (E)-3-(6-(3-dimethylamino-1-(4-tolyl)prop-1E-enyl)-2-pyridyl)acrylic acid (Compound C), was purified by crystallization from isopropanol, m.p. 222-5° (decomp.)

Example 4: (E)-3-(6-(3-Pyrrolidino-1-(4-tolyl)prop-1E-enyl)-2-pyridyl)acrylic acid via the intermediate Ethyl-(E)-3-(6-(4-toluyl)-2-pyridyl)-acrylate.

A mixture of 2-bromo-6-(4-toluoyl)pyridine (56 g), ethyl acrylate (25 mL), triethylamine (30 mL), palladium (II) acetate (0.4 g), triphenylphosphine (0.9 g) and acetonitrile (50 mL) was placed in an autoclave and heated with stirring at 150° for six hours. After cooling the solid product was broken up, washed with water and alcohol, and recrystallized from alcohol, yielding compound 2 (51 g) as colourless prisms mp. 110-12°.

Triphenyl-2-pyrrolidinoethylphosphonium bromide (72 g) was suspended in dry toluene (750 mL) under nitrogen, cooled in ice, and treated during 15 minutes with butyllithium (100 mL, 1.6M in hexane). The bath was removed and stirring continued for six hours. Again with ice cooling compound 2, vide supra, (48 g) dissolved in dry toluene (500 mL) was added during 30 minutes. The mixture was then heated in a bath at 75° for 2 hours. Next day, with ice cooling hydrochloric acid (500 mL, 2M) was added. The aqueous layer was separated, washed with ether, basified with ammonium hydroxide (ice) and extracted with ether. Drying and evaporation gave a mixture of basic esters (46 g). Concentrated sulphuric acid (75 mL) was added and the mixture was plunged into an oil bath at 150° and stirred for 5 minutes. After rapid cooling the mixture was cautiously added to methanol (500 mL). After boiling under reflux for one hour the solution was evaporated to 200 mL in vacuo, poured onto excess ice and basified with ammonium hydroxide. Extraction with ether, drying the washed extracts and evaporation gave a dark oil (39 g). Ethanol (750 mL) and sodium hydroxide solution (150 mL, 1M) were added and the mixture was heated on the steam bath under reduced pressure to remove the alcohols as rapidly as possible. To the remaining aqueous solution sulphuric acid (150 mL, 0.5M) was added and the neutral solution was evaporated to dryness in vacuo. The dry solid remaining was extracted with hot isopropanol (4 x 200 mL). Partial evaporation and cooling gave 19 g of (E)-3-(6-(3-pyrrolidino-1-(4-tolyl)prop-1E-enyl)-2-pyridyl)acrylic acid;m.p. 220-220° (decomp.).

Example 5:(E)-3-(6-Pyrrolidino-1-(4-trifluoromethylphenyl)prop-1E-enyl)-2-pyridyl)acrylic acid via (E)-Ethyl-3-[6-(4-trifluoromethylbenzoyl)-2-pyridyl]-acrylate.

2-Bromo-6-(4-trifluoromethylbenzoyl)pyridine, m.p. 66-68° (prepared from 2,6-dibromopyridine and 4-trifluoromethylbenzonitrile by the method of Ex.1 was converted by the method of Ex.4 to**(E)-Ethyl-3-[6-(4-trifluoromethylbenzoyl)-2-pyridyl]acrylate,** m.p. 129-132°. Further treatment with Wittig reagent by the method of Ex.1 gave, after saponification and crystallisation from isopropanol, (E)-3-(6-(3-pyrrolidino)-(4-trifluoromethylphenyl)prop-1 E-enyl)-2-pyridyl)acrylic acid, m.p. 223-225° (decomp).

Example 6: (E)-3-(6-(3-Pyrrolidino-1-(4-methoxyphenyl)prop-1E-enyl)-2-pyridyl)acrylic acid via (E)-Ethyl-3-(6-(4-methoxybenzoyl)-2-pyridyl)acrylate

4-Methoxybenzonitrile was converted by the method of Ex.5 to 2-bromo-6-(4-methoxybenzoyl)pyridine, m.p. 116-8°, and thence to **(E)-ethyl-3-(6-(4-methoxybenzoyl)-2-pyridyl)acrylate,** m.p. 99-100° and further to (E)-3(6-(3-pyrrolidino-1-(4-methoxyphenyl)prop-1E-enyl)-2-pyridyl)acrylic acid, which formed colourless crystals from isopropanol, m.p. 231-2° (decomp).

Example 7: (E)-3(6-(1-Phenyl-3-pyrrolidinoprop-1E-enyl)-2-pyridyl)acrylic acid via (E)-Ethyl-3-(6-benzoyl-2-pyridyl)-acrylate

By the method of Ex.5 benzonitrile was reacted with 2,6-dibromopyridine to produce 2-bromo-6-benzoylpyridine, m.p. 56-62° which by further processing afforded **(E)-ethyl-3-(6-benzoyl-2-pyridyl)-acrylate**, m.p. 34-36°. Treatment with Wittig reagent then gave (E)3-(6-(1-phenyl-3-pyrrolidinoprop-1E-enyl)-2-pyridyl)acrylic acid which formed white prisms from ethyl acetate, m.p. 180-182° (decomp).

Example 8

(E)-3-(6-(3-Pyrrolidino-1-(4-tolyl)prop-1E-enyl)-2-pyridyl)acrylic acid via the intermediate (E)-Ethyl-3-(6-(4-toluyl)-2-pyridyl)-acrylate.

A solution of vinyl magnesium bromide was prepared under nitrogen from vinyl bromide (5.9 g) and magnesium turnings (1.5 g) in tetrahydrofuran (53 ml). To this ice-cooled solution was added with stirring anhydrous zinc chloride (3.74 g) and the mixture stirred under $N_2$ for 2 hours at or below room temperature. The resultant solution was decanted from the bulk of the insoluble inorganics and gradually added to an ice-cooled and stirred suspension of the keto-ester (7.38 g) in THF (50 ml). The mixture was stirred at room temperature for 44 hours by which time the reaction was almost complete. With cooling 2N aqueous HCl (50 ml) was gradually added and the mixture then poured into water (450 ml) and the product extracted with ether (600 ml) and the extract washed with water (3 x 250 ml) and brine (100 ml) and dried. Filtration and removal of the solvent in vacuo gave ethyl-(E)-3-(6-[1-(4-tolyl)-1-hydroxy-prop-2-enyl]-2-pyridyl)acrylate as a pale brown oil (8.7 g) which contained some residual THF.

To a stirred solution of the above carbinol (8.0 g) in triethylamine (30 ml) was added 4-N,N-dimethylaminopyridine (0.80 g) and then acetic anhydride (8 ml) and the mixture stirred at room temperature overnight. A further aliquot of acetic anhydride (4 ml) was added and the mixture stirred overnight again after which time reaction was essentially complete. With ice-cooling ethanol (30 ml) was added and the mixture stirred at room temperature for 30 min. After concentration in vacuo the residue was dissolved in ether (250 ml) and the solution washed with water (50 ml), saturated sodium bicarbonate solution (2 x 50 ml) water (50 ml) and brine (40 ml) and dried. Filtration and removal of the solvent in vacuo gave a dark red gum (8.4 g). This crude product was then purified by dry-column chromatography using silica (250 g) and eluting with hexane/ether (3:2). Combination of the appropriate fractions and concentration in vacuo gave a product which when triturated with hexane and cooled gave ethyl-(E)-3-6-(1-(4-tolyl)-1-acetoxy-prop-2-enyl)-2-pyridyl acrylate as a pale-cream solid (2.86 g, 31 %) m.pt 99° C. A sample was crystallised from hexane to give white crystals m.pt. 100° C.

The above acetate (1.825 g) was dissolved in acetonitrile (15 ml) and tetrakis triphenylphosphine palladium (0) (60 mg) added followed by triphenylphosphine (25 mg) and then pyrrolidine (0.50 ml). The mixture was then heated at 63-65° C under nitrogen with stirring for 2 hours. After cooling the mixture was poured into water (100 ml) and acifidied by the addition of 2N aqueous hydrochloric acid (20 ml) and the solution extracted with ether (50 ml). The aqueous layer was then neutralised by the addition of a slight excess of aqueous ammonia and the product extracted with ether (100 ml) and the extract washed with water (50 ml) and brine (25 ml) and dried. Filtration and concentration in vacuo gave a pale-brown viscous gum (1.85 g 98%) which was a mixture of steroisomers (E,E:EZ = 58:42) of ethyl-3-(6-(3-pyrrolidino-1-(4-tolyl)prop-1-enyl)-2-pyridyl)acrylate.

This product was mixed with 95% w/w sulphuric acid (5 ml) and heated at 130-135° C with stirring for 1 hour. The resultant solution was cooled and poured into ethanol (50 ml) and the solution heated at reflux for 1 hour. The volume was then reduced to about one half by concentration in vacuo and then water (100 ml) was added and the solution neutralised by the addition of a slight excess of aqueous ammonia. The product was extracted with ether (100 ml) and the extract washed with water (50 ml) and dried. Filtration and concentration in vacuo gave a red gum (1.17 g, 63%) which was predominantly the E,E-isomer ethyl-(E)-3-(6-(3-pyrrolidino-1-(4-tolyl)prop-1E-enyl)-2-pyridyl)acrylate.

The above E,E-ester (0.75 g) was hydrolysed in the usual manner by dissolving in ethanol (15 ml) and adding 1N aqueous sodium hydroxide (3 ml) and removing the alcohol by rotary evaporation in vacuo. To the cooled residue was added 1N aqueous sulphuric acid (3 ml) and the mixture concentrated to dryness in vacuo. The dry residue was extracted with hot isopropanol (3 x 4 ml) and the total extract cooled in the refrigerator to give a white solid (112 mg) which was crystallised from isopropanol (6 ml) to give crystals of the title compound (42 mg).

Example 9

6-(6-(3-Pyrrolidino-1-(4-tolyl)prop-1Z-enyl)-2-pyridyl)hex-5E-enoic acid via the intermediate Methyl-6-(6-(4-toluyl)-2-pyridyl)hex-5-enoate

4-Carboxybutyltriphenylphosphonium bromide (12.8 g) was added under nitrogen to a stirred solution of dimsyl sodium prepared from dimethyl sulphoxide (30 ml) and sodium hydride (2.8 g of 50% oil suspension). After five minutes a solution of 2-(6-formyl-2-pyridyl)-2-(4-tolyl)-1,3-dioxolan (9.9 g) in tetrahydrofuran (20 ml) was added and the mixture was stirred at 45° for 2.5 hours. The cooled mixture was diluted with water (100 ml), washed with ether, acidified with hydrochloric acid (ice) and extracted with chloroform. The extracts were thoroughly washed with water, dried and evaporated to dryness. The residual oil (20 g) was heated in ethanol (100 ml) containing hydrochloric acid (50 ml, 2M) for 40 minutes. The crude acid recovered by evaporation was esterified with methanol/sulphuric acid and the ester was purified by distillation, b.p. 200-210°/0.2 mm. Treatment of this ester with Wittig reagent by the method of Example 1 produced a mixture of acids from which the title compound was isolated by crystallisation from ethyl acetate as off-white prisms, m.p. 118-121°.

**Claims**

Claim for the following Contracting States : BE, CH, DE, FR, GB, IT, LI, LU, NL, SE

1. A compound of the formula (IXa)

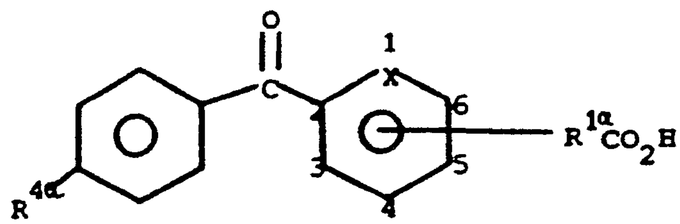

(IXa)

or a salt, ester, or amide thereof, wherein X is nitrogen; the group $R^{1a}CO_2H$ is attached to the 6-position of the pyridine ring; $R^{1a}$ is $(CH_2)_n$ wherein n is an integer 1 to 7, or $(CH_2)_aCH=CH(CH_2)_b$ wherein a and b are independently 0 to 5 and the sum of a and b does not exceed 5; and $R^{4a}$ is hydrogen, halogen, $C_{1-4}$ alkyl, $C_{1-4}$ alkoxy or trifluoromethyl.

Claim for the following Contracting State: AT

1. A process for preparing a compound of the formula (IXa)

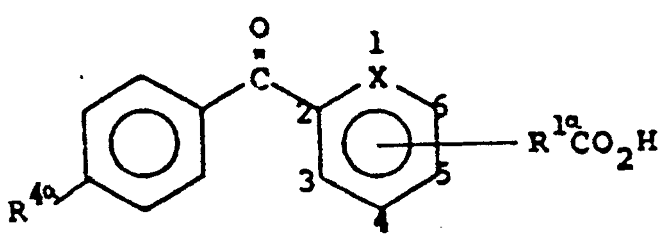

(IXa)

or a salt, ester, or amide thereof, wherein X is nitrogen; the group $R^{1a}CO_2H$ is attached to the 6-position of the pyridine ring; $R^{1a}$ is $(CH_2)_n$ wherein n is an integer 1 to 7, or $(CH_2)_aCH=CH(CH_2)_b$ wherein a and b are independently 0 to 5 and the sum of a and b does not exceed 5; and $R^{4a}$ is hydrogen, halogen, $C_{1-4}$ alkyl, $C_{1-4}$ alkoxy or trifluoromethyl;
the process comprising either
(i) when $R^{1a}$ is trans -CH=CH-, reacting a compound of formula XV

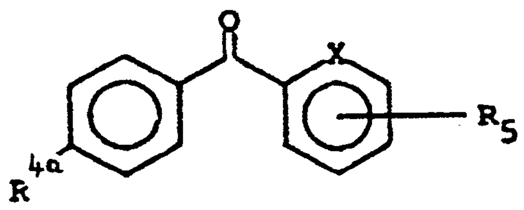

(XV)

wherein $R^{4a}$ is as defined for formula (IXa) and $R_5$ is a halogen atom with an acrylate ester of formula XVI

$CH_2=CHCO_2R_9$ (XVI)

wherein $R_9$ is a $C_{1-4}$ alkyl group;
in the presence of a catalyst consisting of palladium acetate and a triarylphosphine and a tertiary amine at an elevated temperature; or
(ii) reacting a compound for formula XVII

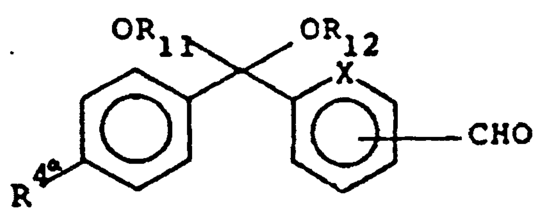

(XVII)

Wherein $R^{4a}$ and X are as defined for formula (IXa) and $R_{11}$ and $R_{12}$ may be the same or different and are each $C_{1-4}$ alkyl or may together form a cyclic ketal containing up to 6 carbon atoms,
with malonic acid in the presence of a suitable base, or with a Wittig reagent preparable by treating a phosphonium salt XVIIIA or a phosphonate ester XVIIIB with a suitable base in an appropriate solvent

$$(R_9)_3P^+(CH_2)_dCOR_6Hal^-$$

(XVIIIA)

$$(R_{10})_2PO(CH_2)dCOR_6$$

(XVIIIB)

wherein Hal represents chlorine or bromine, $COR_6$ is an acid, ester or amide group, $R_9$ is a $C_{1-4}$ alkyl or phenyl group and $R_{10}$ is a $C_{1-4}$ alkoxy group and d is an integer of 1 to 6.

**Revendications**

Revendication pour les Etats contractants suivants : BE, CH, DE, FR, GB, IT, LI, LU, NL, SE

**1.** Composé de formule (IXa)

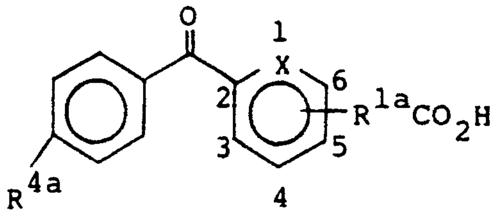

(IXa)

ou un sel, ester ou amide de celui-ci,
où X est azote;
le radical $R^{1a}CO_2H$ est fixé à la position 6 du cycle pyridine;
$R^{1a}$ est $(CH_2)_n$ où n est un entier de 1 à 7 ou bien $(CH_2)_aCH=CH(CH_2)_b$ où a et b sont indépendamment 0 à 5 et la somme de a et b n'excède pas 5; et
$R^{4a}$ est hydrogène, halogène, $C_{1-4}$-alcoyle, $C_{1-4}$-alcoxy ou trifluorométhyle.

Revendication pour l'Etat contractant suivant : AT

1. Procédé de préparation d'un composé de formule (IXa)

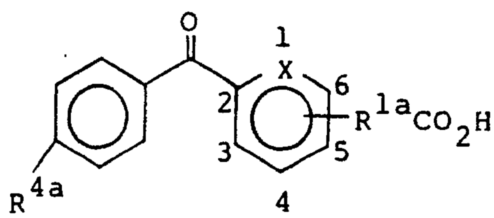

(IXa)

ou d'un sel, ester ou amide de celui-ci,
où X est azote;
le radical $R^{1a}CO_2H$ est fixé à la position 6 du cycle pyridine;
$R^{1a}$ est $(CH_2)_n$ où n est un entier de 1 à 7 ou bien $(CH_2)_aCH=CH(CH_2)_b$ où a et b sont indépendamment 0 à 5 et la somme de a et b n'excède pas 5; et
$R^{4a}$ est hydrogène, halogène, $C_{1-4}$-alcoyle, $C_{1-4}$-alcoxy ou trifluorométhyle;
lequel procédé comprend
(i) lorsque $R^{1a}$ est <u>trans</u> -CH=CH-, la réaction d'un composé de formule (XV)

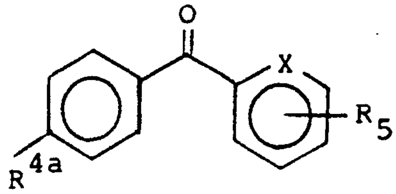

(XV)

où $R^{4a}$ est tel que défini à propos de la formule (IXa) et $R_5$ est un atome d'halogène, avec un ester acrylique de formule (XVI)

$CH_2 = CHCO_2R_9$ (XVI)

où $R_9$ est un radical $C_{1-4}$-alcoyle;
en présence d'un catalyseur consistant en acétate de palladium et en une triarylphosphine et une amine tertiaire, à une température élevée; ou bien
(ii) la réaction d'un composé de formule (XVII)

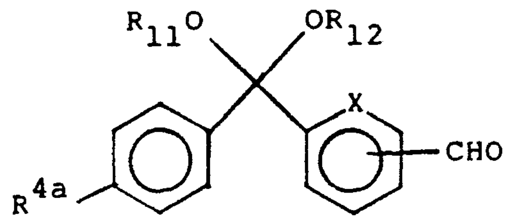

(XVII)

où $R^{4a}$ et X sont tels que définis à propos de la formule (IXa) et $R_{11}$ et $R_{12}$ peuvent être identiques ou différents et sont chacun $C_{1-4}$-alcoyle ou peuvent former ensemble un cétal cyclique comptant jusqu'à 6 atomes de carbone,
avec l'acide malonique en présence d'une base appropriée ou avec un réactif de Wittig qui peut être préparé en faisant réagir un sel de phosphonium XVIIIA ou un ester phosphonique XVIIIB avec une base appropriée dans un solvant convenable

$$(R_9)_3P^+(CH_2)_dCO_2R_6\ Hal^- \qquad (R_{10})_2PO(CH_2)_dCO_2R_6$$

(XVIII A) (XVIII B)

où Hal représente chlore ou brome, $COR_6$ est un radical acide, ester ou amide, $R_9$ est un radical $C_{1-4}$-alcoyle ou phényle, $R_{10}$ est un radical $C_{1-4}$-alcoxy et d est un entier de 1 à 6.

**Ansprüche**

Patentanspruch für folgende Vertragsstaaten : BE, CH, DE, FR, GB, IT, LI, LU, NL, SE

1. Verbindung der Formel (IXa)

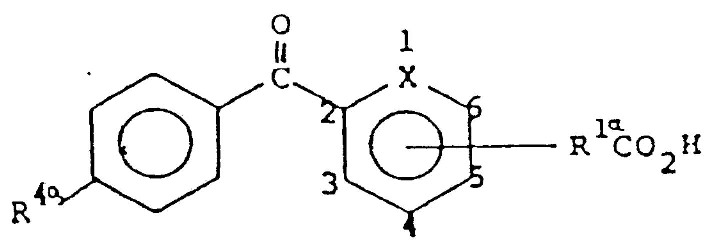

(IXa)

oder ein Salz, Ester oder Amid hievon, worin X Stickstoff bedeutet; die Gruppe $R^{1a}CO_2H$ sich in Stellung 6 des Pyridinringes befindet; $R^{1a}$ die Bedeutung $(CH_2)_n$, wobei n eine ganze Zahl von 1 bis 7 ist, oder $(CH_2)_aCH=CH(CH_2)_b$, wobei a und b unabhängig Null bis 5 sind und die Summe von a und b 5 nicht übersteigt, hat; und $R^{4a}$ Wasserstoff, Halogen, $C_1$-$C_4$-Alkyl, $C_1$-$C_4$-Alkoxy oder Trifluormethyl darstellt.

Patentanspruch für folgenden Vertragsstaat : AT

1. Verfahren zum Herstellen einer Verbindung der Formel (IXa)

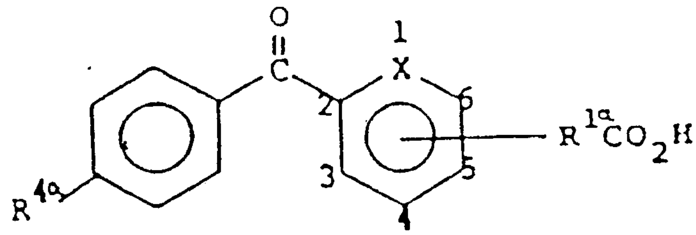

(IXa)

oder eines Salzes, Esters oder Amids hievon, worin X Stickstoff bedeutet; die Gruppe $R^{1a}CO_2H$ sich in Stellung 6 des Pyridinringes befindet; $R^{1a}$ die Bedeutung $(CH_2)_n$, wobei n eine ganze Zahl von 1 bis 7 ist, oder $(CH_2)_aCH=CH(CH_2)_b$, wobei a und b unabhängig Null bis 5 sind und die Summe von a und b 5 nicht übersteigt, hat; und $R^{4a}$ Wasserstoff, Halogen, $C_1$-$C_4$-Alkyl, $C_1$-$C_4$-Alkoxy oder Trifluormethyl darstellt, welches Verfahren entweder

(i) wenn $R^{1a}$ trans-CH=CH- ist, das Umsetzen einer Verbindung der Formel (XV)

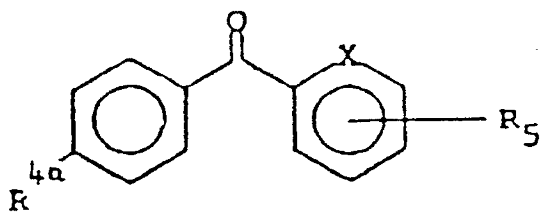

(XV),

worin $R^{4a}$ wie für Formel (IXa) definiert ist und $R_5$ ein Halogenatom darstellt, mit einem Acrylatester der Formel (XVI)

$CH_2=CHCO_2R_9$ (XVI),

worin $R_9$ eine $C_1$-$C_4$-Alkylgruppe bedeutet, in Anwesenheit eines Katalysators bestehend aus Palladiumacetat und einem Triarylphosphin und einem tertiären Amin bei erhöhter Temperatur, oder (ii) das Umsetzen einer Verbindung der Formel (XVII)

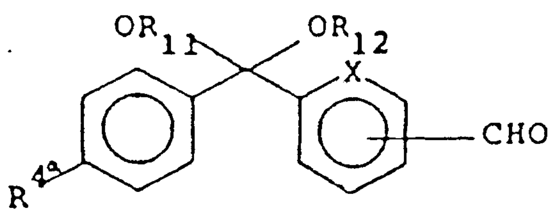

(XVII),

worin $R^{4a}$ und X wie für Formel (IXa) definiert sind und $R_{11}$ und $R_{12}$ gleich oder verschieden sein können und jeweils $C_1$-$C_4$-Alkyl bedeuten oder miteinander ein cyclisches Ketal mit bis zu 6 Kohlenstoffatomen bilden können, mit Malonsäure in Anwesenheit einer geeigneten Base oder mit einem Wittig-Reagens, das durch Behandlung eines Phosphoniumsalzes (XVIIIA)

$(R_9)_3P^+(CH_2)_dCOR_6Hal^-$ (XVIIIA)

oder eines Phosphonatesters (XVIIIB)

$(R_{10})_2PO(CH_2)_dCOR_6$ (XVIIIB),

worin Hal Chlor oder Brom bedeutet, $COR_6$ eine Säure-, Ester- oder Amidgruppe darstellt, $R_9$ eine $C_1$-$C_4$-Alkyl- oder Phenylgruppe ist, $R_{10}$ eine $C_1$-$C_4$-Alkoxygruppe bedeutet und d eine ganze Zahl von 1 bis 6 ist, mit einer geeigneten Base in einem geeigneten Lösungsmittel herstellbar ist, umfaßt.